# EUROPÄISCHE PATENTSCHRIFT

(11) **EP 0 492 249 B1**
(45) Veröffentlichungstag und Bekanntmachung des Hinweises auf die Patenterteilung: **22.05.1996**
(21) Anmeldenummer: 91121066.4
(22) Anmeldetag: 09.12.1991
(51) Int. Cl.: C07D 271/06, A61K 31/41, A01N 43/82

(54) **Verwendung von substituierten 1,2,4-Oxadiazolderivaten zur Bekämpfung von Endoparasiten, neue substituierte 1,2,4-Oxadiazolderivate und Verfahren zu ihrer Herstellung**
Use of substituted 1,2,4-oxadiazole derivatives for controlling endoparasites, new 1,2,4-oxadiazole derivatives and process for their preparation
Utilisation de dérivés de 1,2,4-oxadiazole pour lutter contre les endoparasites, nouveaux dérivés de 1,2,4-oxadiazole et procédé pour leur préparation

(30) Priorität: 22.12.1990 DE 4041474
(43) Veröffentlichungstag der Anmeldung: 01.07.1992
(73) Patentinhaber: BAYER AG, 51368 Leverkusen (DE)
(72) Erfinder: Jeschke, Peter, Dr., W-5090 Leverkusen 1 (DE); Lindner, Werner, Dr., W-5000 Köln 80 (DE); Harder, Achim, Dr., W-5000 Köln 80 (DE); Mencke, Norbert, Dr., W-5090 Leverkusen 3 (DE); Haberkorn, Axel, Prof. Dr., W-5600 Wuppertal 1 (DE)

(56) Entgegenhaltungen:
- GB-A- 1 228 142
- GB-A- 1 357 733
- US-A- 4 012 377
- JOURNAL OF THE CHEMICAL SOCIETY, PERKIN TRANSACTIONS 1 1973, LONDON GB Seiten 2241 - 2249; J.A. CLAISSE ET AL.: 'Some 5-Unsubstituted Acetylenic and Vinylic 1,2,4-Oxadiazoles'

## Beschreibung

Die vorliegende Erfindung betrifft neue substituierte 1,2,4-Oxadiazolderivate, Verfahren zu ihrer Herstellung und ihre Verwendung als Endoparasitizide.

Es ist bereits bekannt, daß bestimmte 1,2,4-Oxadiazole, wie beispielsweise die Verbindung (E)-3-Styryl-1,2,4-oxadiazol parasitizide Wirksamkeit besitzen (vgl. z.B. US-Pat. 4 012 377). Weitere substituierte Styryl-1,2,4-oxadiazole sind bekannt aus GB-A 1 357 733 und GB-A 1 228 142.

Die Wirksamkeit dieser vorbekannten Verbindungen ist jedoch bei niedrigen Aufwandmengen und Konzentrationen nicht völlig zufriedenstellend.

Gegenstand der vorliegenden Anmeldung sind:
1. Neue substituierte 1,2,4-Oxadiazolderivate der allgemeinen Formel (I) und ihre Stereoisomere 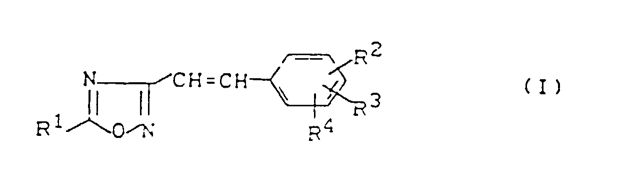 in welcher
   - R¹: für Wasserstoff, C₁₋₆-Alkyl, das gegebenenfalls substituiert ist durch 1 bis 6 Halogenatome, C₃₋₆-Cycloalkyl, C₁₋₆-Alkoxy, Hydroxy, gegebenenfalls substituiertes Phenyl, C₁₋₄-Alkyl-carbonyl, C₁₋₆-Alkoxycarbonyl sowie für C₃₋₆-Cycloalkyl steht,
   - R: für Wasserstoff, Halogen, CN, NO₂, C₁₋₆-Alkyl, 1 bis 6 Halogen-C₁₋₆-Alkyl, C₁₋₆-Alkoxy oder C₁₋₆-Thioalkyl steht,
   - R³: für einen der bei R angegebenen Reste steht,
   - R⁴: für SH, 1 bis 5 Halogen-C₁₋₆-alkoxy, 1 bis 6 Halogen-C₁₋₄-alkylthio, 1 bis 6 Halogen-C₁₋₆-alkylsulfinyl, 1 bis 5 Halogen-C₁₋₆-alkylsulfonyl, gegebenenfalls substituiertes Phenoxy, gegebenenfalls substituiertes Phenylmercapto, gegebenenfalls substituiertes Phenyl-C₁₋₆-alkoxy, gegebenenfalls substituiertes Phenyl-C₁₋₆-alkylthio, C₁₋₂-Alkylendioxy, 1 bis 4 Halogen-C₁₋₂-alkylendioxy, C₁₋₆-Alkylamino, Di-C₁₋₄-alkylamino, Acetylamino, Benzolsulfonylamino, -CONH₂, -CONH(C₁₋₄-Alkyl), C₁₋₄-Alkoxycarbonyl, C₁₋₄-Alkylcarbonyl, C₁₋₄-Alkylcarbonyloxy, C₁₋₄-Alkoxy-C₁₋₄-alkyl, C₁₋₄-Alkyl-C₁₋₄-alkoxy und Hydroxy-C₁₋₄-alkoxy steht,
   sowie für den Fall, daß mindestens einer der Reste R und R³ eine andere Bedeutung als Wasserstoff hat, zusätzlich für Halogen, C₁₋₄-Alkyl, 1 bis 6 Halogen-C₁₋₄-alkyl, C₁₋₄-Alkoxy und C₁₋₄-Alkylthio OH steht, sowie für den Fall, daß R¹ für gegebenenfalls substituiertes C₁₋₆-Alkyl steht, zusätzlich für C₁₋₄-Alkoxy und NH₂ steht.

   Als mögliche Substituenten der gegebenenfalls substituierten Reste seien genannt:
   Halogen, C₁-C₄-Alkyl, C₁-C₄-Halogenalkyl, C₁-C₄-Alkoxy, C₁-C₄-Halogenalkoxy, C₁-C₄-Halogenalkylthio, C₁-C₄-Halogenalkylsulfinyl, C₁-C₄-Halogenalkylsulfonyl, Methylendioxy oder Ethylendioxy, die gegebenenfalls durch Fluor oder Chlor substituiert sind, Amino, C₁-C₄-Alkyl- und Dialkylamino, Acetylamino.
2. Verfahren zur Herstellung der substituierten 1,2,4-Oxadiazolderivate der Formel (I) und deren Stereoisomere, 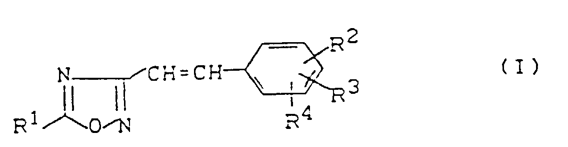 in welcher
   - R¹: für Wasserstoff, C₁₋₆-Alkyl, das gegebenenfalls substituiert ist durch 1 bis 6 Halogenatome, C₃₋₆-Cycloalkyl, C₁₋₆-Alkoxy, Hydroxy, gegebenenfalls substituiertes Phenyl, C₁₋₄-Alkyl-carbonyl, C₁₋₆-Alkoxycarbonyl sowie für C₃₋₆-Cycloalkyl steht,
   - R: für Wasserstoff, Halogen, CN, NO₂, C₁₋₆-Alkyl, 1 bis 6 Halogen-C₁₋₆-Alkyl, C₁₋₆-Alkoxy oder C₁₋₆-Thioalkyl steht,
   - R³: für einen der bei R angegebenen Reste steht,
   - R⁴: für SH, 1 bis 5 Halogen-C₁₋₆-alkoxy, 1 bis 6 Halogen-C₁₋₄-alkylthio, 1 bis 6 Halogen-C₁₋₆-alkylsulfinyl, 1 bis 5 Halogen-C₁₋₆-alkylsulfonyl, gegebenenfalls substituiertes Phenoxy, gegebenenfalls substituiertes Phenylmercapto, gegebenenfalls substituiertes Phenyl-C₁₋₆-alkoxy, gegebenenfalls substituiertes Phenyl-C₁₋₆-alkylthio, C₁₋₂-Alkylendioxy, 1 bis 4 Halogen-C₁₋₂-alkylendioxy, C₁₋₆-Alkylamino, Di-C₁₋₄-alkylamino, Acetylamino, Benzolsulfonylamino, -CONH₂, -CONH(C₁₋₄-Alkyl), C₁₋₄-Alkoxycarbonyl, C₁₋₄-Alkylcarbonyl, C₁₋₄-Alkylcarbonyloxy, C₁₋₄-Alkoxy-C₁₋₄-alkyl, C₁₋₄-Alkyl-C₁₋₄-alkoxy und Hydroxy-C₁₋₄-alkoxy steht,
   sowie für den Fall, daß mindestens einer der Reste R und R³ eine andere Bedeutung als Wasserstoff hat, zusätzlich für Halogen, C₁₋₄-Alkyl, bis 6 Halogen-C₁₋₄-alkyl,C₁₋₄-Alkoxy und C₁₋₄-Alkylthio OH steht, sowie für den Fall, daß R¹ für gegebenenfalls substituierters C₁₋₆-Alkyl steht, zusätzlich für C₁₋₄-Alkoxy und NH₂ steht,

Als mögliche Substituenten der gegebenenfalls substituierten Reste seien genannt:
Halogen, C₁-C₄-Alkyl, C₁-C₄-Halogenalkyl, C₁-C₄-Alkoxy, C₁-C₄-Halogenalkoxy, C₁-C₄-Halogenalkylthio, C₁-C₄-Halogenalkylsulfinyl, C₁-C₄-Halogenalkylsulfonyl, Methylendioxy oder Ethylendioxy, die gegebenenfalls durch Fluor oder Chlor substituiert sind, Amino, C₁-C₄-Alkyl- und Dialkylamino, Acetylamino,
dadurch gekennzeichnet, daß man ein Amidoximderivat der Formel (II) und Stereoisomere davon 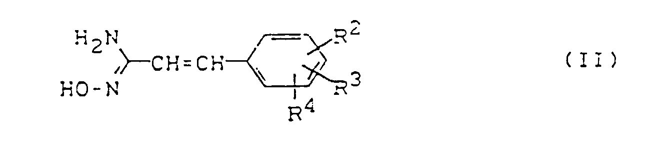 in welcher
R, R³ und R⁴ die oben angegebenen Bedeutungen besitzen,
a) mit einem Carbonsäureorthoester der Formel (III)

   R¹-C(O-A)₃ (III)

   in welcher
   - R¹: die oben angegebene Bedeutung besitzt und
   - A: für Alkyl, insbesondere für Methyl oder Ethyl steht,
   umsetzt oder
b) mit einem Carbonsäureester der Formel (IV) 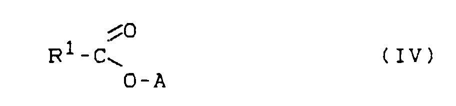 in welcher
   - R¹: und A die oben angegebene Bedeutung haben
   umsetzt oder
c) mit Carbonsäureanhydriden der Formel (V)

   (R¹-CO)₂O (V)

   in welcher
   - R¹: die oben angegebene Bedeutung besitzt
   umsetzt oder
d) mit einem Carbonsäurehalogenid der Formel (VI) 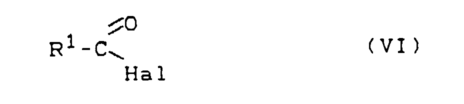 in welcher
   - R¹: die oben angegebene Bedeutung hat und
   - Hal: für Halogen, wie Fluor, Chlor oder Brom, vorzugsweise für Chlor steht,
   in Gegenwart eines Verdünnungsmittels und in Gegenwart eines Reaktionshilfsmittels umsetzt.

Die Verbindungen der Formel (I) lassen sich hervorragend als Endoparasitizide insbesondere auf dem Gebiet der Veterinärmedizin einsetzen.

Überraschenderweise zeigen die erfindungsgemäßen substituierten 1,2,4-Oxadiazolderivate der allgemeinen Formel (I) eine erheblich bessere Wirksamkeit gegenüber Endoparasiten, als die aus dem Stand der Technik bekannten 1,2,4-Oxadiazole, wie beispielsweise die Verbindung (E)-3-Styryl-1,2,4-oxadiazol oder (E)-3-(4-Chlor-styryl)-1,2,4-oxadiazol welche strukturell und wirkungsmäßig naheliegende Verbindungen sind (vgl. z.B. US-Pat. 4 012 377).

Die erfindungsgemäßen substituierten 1,2,4-Oxadiazolderivate sind durch die Formel (I) allgemein definiert.

Bevorzugt sind Verbindungen der Formel (I), in welcher
- R¹: für Wasserstoff, C₁₋₆-Alkyl, das gegebenenfalls substituiert ist durch 1 bis 6 Halogenatome wie Fluor oder Chlor, C₃₋₆-Cycloalkyl, C₁₋₆-Alkoxy, Hydroxy, gegebenenfalls substituiertes Phenyl, C₁₋₄-Alkylcarbonyl, C₁₋₆-Alkoxycarbonyl sowie für C₃₋₆-Cycloalkyl insbesondere Cyclopropyl, Cyclopentyl oder Cyclohexyl steht,
- R: für Wasserstoff, Halogen insbesondere Fluor, Chlor oder Brom, CN, NO₂, C₁₋₆-Alkyl, 1 bis 6 Halogen-C₁₋₆-alkyl insbesondere Trifluormethyl, Trichlormethyl oder Fluor-chlorethylen, C₁₋₆-Alkoxy insbesondere Methoxy, Ethoxy oder Isopropoxy, oder C₁₋₆-Thioalkyl insbesondere Methylthio steht,
- R³: für einen der bei R angegebenen Reste steht,
- R⁴: für SH, 1 bis 5 Halogen-C₁₋₆-alkoxy, 1 bis 6 Halogen-C₁₋₄-alkylthio, 1 bis 6 Halogen-C₁₋₆-alkylsulfinyl, 1 bis 5 Halogen-C₁₋₆-alkylsulfonyl, gegebenenfalls substituiertes Phenoxy, gegebenenfalls substituiertes Phenylmercapto, gegebenenfalls substituiertes Phenyl-C₁₋₆-alkoxy, gegebenenfalls substituiertes Phenyl-C₁₋₆-alkylthio, C₁₋₂-Alkylendioxy, 1 bis 4 Halogen-C₁₋₂-alkylendioxy, C₁₋₆-Alkylamino, Di-C₁₋₄-alkylamino, Acetylamino, Benzol-sulfonylamino, -CONH₂, -CONH(C₁₋₄-Alkyl), C₁-₄-Alkoxycarbonyl, C₁₋₄-Alkylcarbonyl, C₁₋₄-Alkylcarbonyloxy, C₁₋₄-Alkoxy-C₁₋₄-alkyl, C₁₋₄-Alkyl-C₁₋₄-alkoxy und Hydroxy-C₁₋₄-alkoxy steht,
sowie für den Fall, daß mindestens einer der Reste R und R³ eine andere Bedeutung als Wasserstoff hat, zusätzlich für Halogen, C₁₋₄-Alkyl, 1 bis 6 Halogen-C₁₋₄-alkyl,C₁₋₄-Alkoxy und C₁₋₄-Alkylthio, OH steht, sowie für den Fall, daß R¹ für gegebenenfalls substituiertes C₁₋₆-Alkyl steht, zusätzlich für C₁₋₄-Alkoxy und NH₂ steht,
sowie für den Fall, daß R¹ für gegebenenfalls substituiertes C₁₋₆-Alkyl steht, zusätzlich für C₁₋₄-Alkoxy steht.

Als mögliche Substituenten der gegebenenfalls substituierten Reste seien genannt:
Halogen, insbesondere Fluor, Chlor oder Brom,; C₁-C₄-Alkyl, insbesondere Methyl, C₁-C₄-Halogenalkyl, insbesondere Trifluormethyl, C₁-C₄-Alkoxy, insbesondere Methoxy, Isopropyloxy, C₁-C₄-Halogenalkoxy, insbesondere Trifluormethoxy, Difluormethoxy, Fluorchlorethoxy, Hexafluorpropyloxy, C₁-C₄-Halogenalkylthio, insbesondere Trifluormethylthio, Fluorchlormethylthio, C₁-C₄-Halogenalkylsulfinyl, insbesondere Trifluormethylsulfinyl, C₁-C₄-Halogenalkylsulfonyl, insbesondere Trifluormethylsulfonyl, Methylendioxy oder Ethylendioxy, die gegebenenfalls durch Fluor oder Chlor substituiert sind, Amino, C₁-C₄-Alkyl- und Dialkylamino, Acylamino insbesondere Acetylamino.

Ganz besonders bevorzugt sind Verbindungen der Formel (I), in welcher
- R¹: für Wasserstoff, C₁₋₆-Alkyl, das gegebenenfalls substituiert ist durch 1 bis 6 Halogenatome wie Fluor oder Chlor, C₃₋₆-Cycloalkyl, C₁₋₆-Alkoxy, Hydroxy, gegebenenfalls substituiertes Phenyl, C₁₋₄-Alkylcarbonyl, C₁₋₆-Alkoxycarbonyl sowie für C₃₋₆-Cycloalkyl insbesondere Cyclopropyl, Cyclopentyl oder Cyclohexyl steht,
- R: für Wasserstoff, Halogen insbesondere Fluor, Chlor oder Brom, CN, NO₂, C₁₋₆-Alkyl, 1 bis 6 Halogen-C₁₋₆-alkyl insbesondere Trifluormethyl, Trichlormethyl oder Fluor-chlorethylen, C₁₋₆-Alkoxy insbesondere Methoxy, Ethoxy oder Isopropoxy, oder C₁₋₆-Thioalkyl insbesondere Methylthio steht,
- R³: für Halogen insbesondere Fluor, Chlor oder Brom, CN, NO₂, C₁₋₆-Alkyl, 1 bis 6 Halogen-C₁₋₆-alkyl insbesondere Trifluormethyl, Trichlormethyl oder Fluor-chlorethylen, C₁₋₆-Alkoxy insbesondere Methoxy, Ethoxy oder Isopropoxy, oder C₁₋₆-Thioalkyl insbesondere Methylthio steht,
- R⁴: für SH, 1 bis 5 Halogen-C₁₋₆-alkoxy, 1 bis 5 Halogen-C₁₋₄-alkylthio, 1 bis 6 Halogen-C₁₋₆-alkylsulfinyl, 1 bis 5 Halogen-C₁₋₆-alkylsulfonyl, gegebenenfalls substituiertes Phenoxy, gegebenenfalls substituiertes Phenylmercapto, gegebenenfalls substituiertes Phenyl-C₁₋₆-alkoxy, gegebenenfalls substituiertes Phenyl-C₁₋₆-alkylthio, C₁₋₂-Alkylendioxy, 1 bis 4 Halogen-C₁₋₂-alkylendioxy, C₁₋₆-Alkylamino, Di-C₁₋₄-alkylamino, Acylamino wie insbesondere Acetylamino, oder Benzolsulfonylamino, Carbamoyl wie insbesondere die Reste -CONH₂, CONH(C₁₋₄-Alkyl) oder C₁₋₄,
Alkoxycarbonyl, C₁₋₄-Alkylcarbonyl, C₁₋₄-Alkylcarbonyloxy, C₁₋₄-Alkoxy-C₁₋₄-alkyl , C₁₋₄-Alkyl-C₁₋₄-alkoxy und Hydroxy-C₁₋₄-alkoxy steht,
sowie zusätzlich für Halogen wie Fluor, Chlor oder Brom, C₁₋₄-Alkyl, 1 bis 6 Halogen-C₁₋₄-alkyl, C₁₋₄-Alkoxy und C₁₋₄-Alkylthio OH steht.

Besonders hervorgehoben seien Verbindungen der Formel (I), in welcher
- R¹: die obengenannte Bedeutung hat;
- R: für Wasserstoff steht,
- R³: für Fluor oder Chlor steht,
- R⁴: für Fluor oder Chlor steht.

Weiter seien besonders hervorgehoben Verbindungen der Formel (I), in welcher
- R¹: für gegebenenfalls substituiertes Alkyl steht,
- R und R³: die obengenannte Bedeutung haben,
- R⁴: für C₁₋₄-Alkoxy insbesondere Methoxy steht.

Im einzelnen seien folgende Verbindungen (I) genannt, in welcher die Reste R¹, R, R³ und R⁴ die angegebene Bedeutung haben.

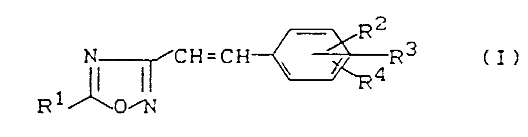

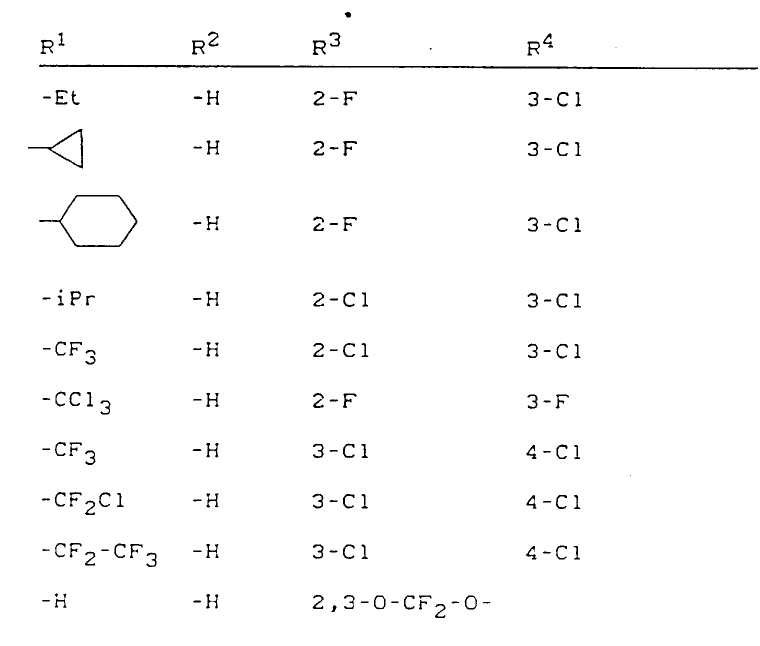

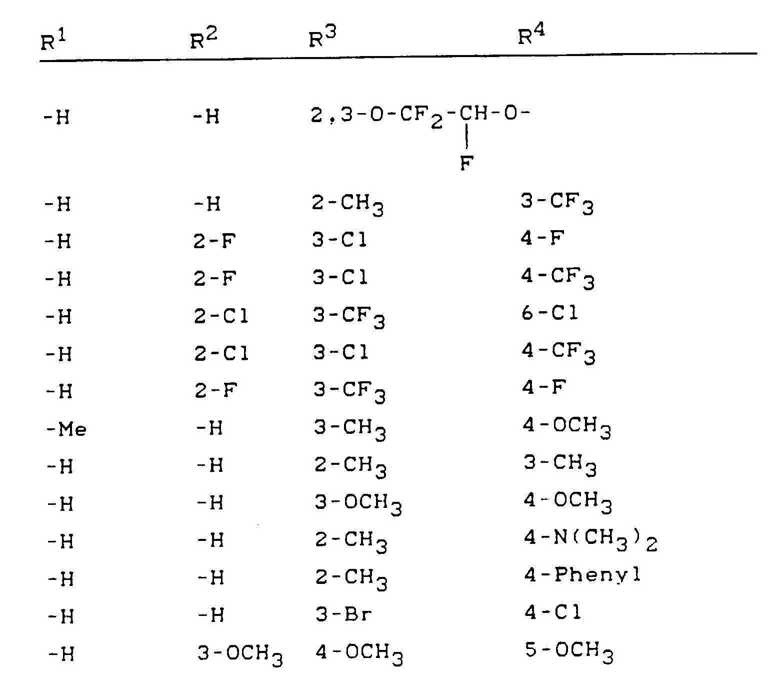

Die Verbindungen der Formel (I) sind neu, sie lassen sich nach den oben unter 2) angegebenen Verfahren a) bis d) herstellen (vgl. z.B. US-Pat. 4 012 377; Claisse et al. J. Chem. Soc. Perkin, Trans. I, 20 (1973), S. 2241-2249).

Setzt man bei Verfahren 2a) zur Herstellung der neuen substituierten 1,2,4-Oxadiazolderivate als Verbindung der Formel (II) (E)-4-Chlor-3-trifluormethyl-zimtsäureamidoxim und als Verbindung der Formel (III) Orthoameisensäuretriethylester ein, so läßt sich das Verfahren durch folgendes Reaktionsschema wiedergeben.

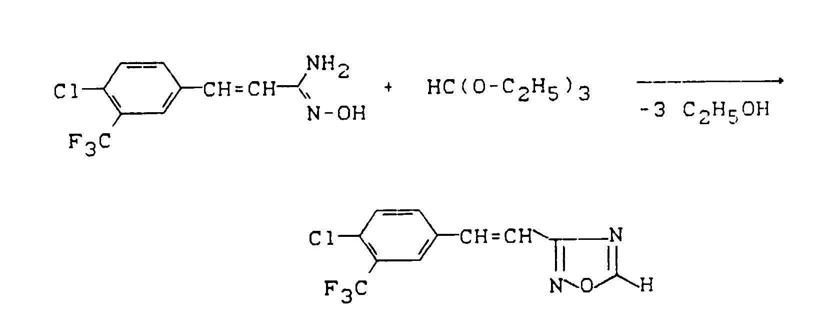

Die zur Durchführung des erfindungsgemäßen Verfahrens 2a) als Ausgangsstoffe benötigten Amidoxime sind durch die Formel (II) allgemein definiert. In dieser Formel stehen R, R³ und R⁴ vorzugsweise für diejenigen Reste, die bereits im Zusammenhang mit der Beschreibung der erfindungsgemäßen Stoffe der Formel (I) als bevorzugt für diese Substituenten genannt wurden.

Die als Ausgangsmaterialien verwendeten Verbindungen der Formel (II) sind teilweise bekannt (vgl. z.B. Claisse et al. J Chem. Soc. Perkin, Trans. I 20 (1973), S. 2241 bis 2249; Yale et al. J. Heterocycl. Chem. 15(8), S. 1373 bis 1378) bzw. können nach den dort beschriebenen Verfahren erhalten werden.

Die Verbindungen der Formel (II) können als geometrische Isomere oder Isomerengemische unterschiedlicher Zusammensetzung vorliegen.

Die außerdem für die Durchführung des erfindungsgemäßen Verfahrens 2a) als Ausgangsstoffe zu verwendenden Carbonsäureorthoester sind allgemein durch die Formel (III) definiert In dieser Formel (III) hat R¹ die Bedeutungen die bereits im Zusammenhang mit der Beschreibung der erfindungsgemäßen Stoffe der Formel (I) als bevorzugt für diesen Substituenten genannt wurden. Die Carbonsäureorthoester der Formel (III) sind allgemein bekannte Verbindungen der organischen Chemie.

Im einzelnen seien folgende Verbindungen der Formel (II) genannt:

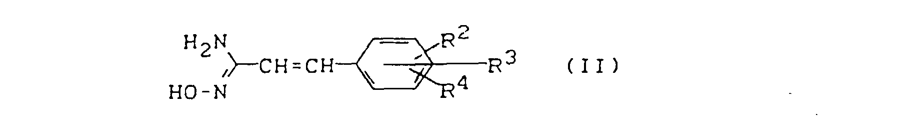

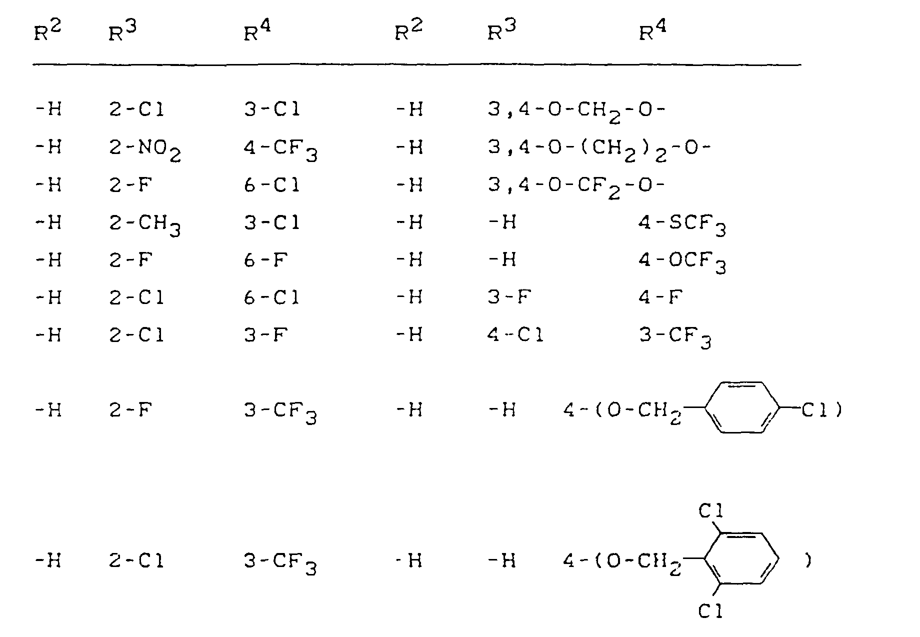

Die Verbindungen der Formel (II) und (III) werden vorzugsweise in Gegenwart eines sauren Katalysators umgesetzt. Als solche kommen dabei praktisch alle Mineralsäuren oder Lewis-Säuren in Frage. Zu den Mineralsäuren gehören vorzugsweise Halogenwasserstoffsäuren wie Fluorwasserstoffsaure, Chlorwasserstoffsäure, Bromwasserstoffsäure oder Iodwasserstoffsäure sowie Schwefelsäure, Phosphorsäure, Phosphorige Säure, Salpetersäure und zu den Lewis-Säuren gehören vorzugsweise Aluminiumchlorid, Bortrifluorid oder sein Etherat, Titan(IV)-chlorid, Zinn(IV)-chlorid.

Besonders bevorzugt werden folgende Lewis-Säuren eingesetzt:
Bortrifluorid oder sein Etherat, Aluminiumchlorid.

Das Verfahren 2a) wird durchgeführt, indem man Verbindungen der Formel (II) mit einem Überschuß der Verbindungen der Formel (III) zusammengibt und in Gegenwart eines sauren Katalysators erhitzt. Hierbei ist die Verbindung (III) zugleich Verdünungsmittel. Die Reaktionsdauer beträgt ca. 1 bis 4 Stunden. Die Reaktion wird bei Temperaturen zwischen +20°C und +200°C, bevorzugt zwischen +100°C und +155°C durchgeführt. Vorzugsweise arbeitet man bei dem Druck, der sich beim Erhitzen auf die erforderliche Reaktionstemperatur unter den Reaktionsbedingungen einstellt.

Nach vollendeter Umsetzung wird das Reaktionsgemisch abgekühlt, im Vakuum eingeengt, der verbleibende Rückstand in einem organischen Lösungsmittel aufgenommen und in an sich bekannter Weise aufgearbeitet. Die anfallenden Produkte lassen sich in üblicher Weise durch Umkristallisieren, Vakuumdestillation oder Säulenchromatographie reinigen (vgl. auch die Herstellungsbeispiele).

Alternativ kann diese Umsetzung auch mit einem Meerwein-Reagenz (z.B. einem Dialkylacetal von Dimethylformamid) oder dem Vilsmeier-Haack-Reagenz (POCl₃, N,N-Dimethylformamid) durchgeführt werden.

Setzt man bei Verfahren 2b) als Verbindung der Formel (II) (E)-3-Chlor-2-fluor-zimtsäureamidoxim und als Verbindung der Formel (IV) Acetessigsäureethylester ein, läßt sich das Verfahren durch folgendes Reaktionsschema beschreiben:

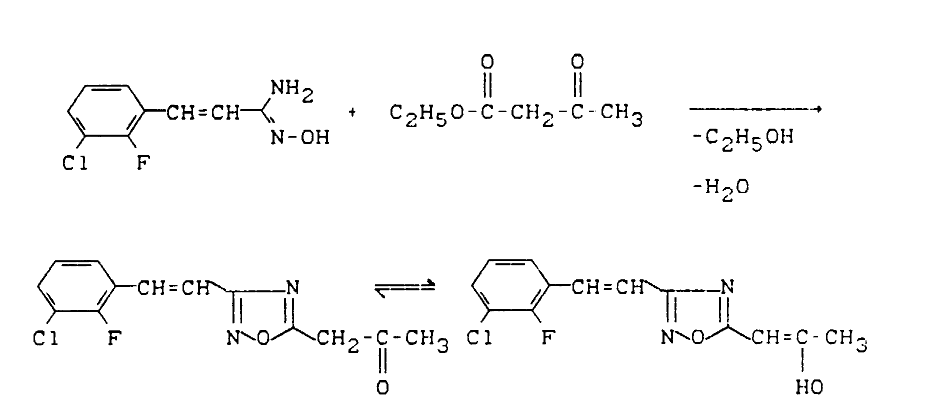

Die Verbindungen der Formel (I) können in Form ihrer verschiedenen Tautomeren (Keto/Enol) sowie als Gemisch dieser Tautomeren unterschiedlicher Zusammensetzung vorliegen.

Bevorzugt werden bei Verfahren 2b) die Verbindungen der Formel (II) eingesetzt, bei denen die Reste R, R³ und R⁴ die bei den Verbindungen der Formel (I) genannten bevorzugten und besonders bevorzugten Bedeutungen besitzen. Die außerdem für die Durchführung des erfindungsgemäßen Verfahrens 2b) als Ausgangsstoffe zu verwendenden Carbonsäureester sind durch die Formel (IV) allgemein definiert. Die Carbonsäureester der Formel (IV) sind allgemein bekannte Verbindungen der organischen Chemie.

Die Reaktion wird vorzugsweise unter Verwendung von Verdünnungsmitteln durchgeführt. Als Verdünnungsmittel zur Durchführung des erfindungsgemäßen Verfahrens 2b) kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Ether wie Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Dichlordiethylether; Nitrokohlenwasserstoffe wie Nitromethan, Nitroethan, Nitrobenzol, Chlornitrobenzol, o-Nitrotoluol; Nitrile wie Acetonitril, Butyronitril, Isobutyronitril, Benzonitril, m-Chlorbenzonitril; aliphatische, cycloaliphatische oder aromatische Kohlenwasserstoffe wie Heptan, Hexan, Nonan, Cymol, Benzinfraktionen innerhalb eines Siedepunktintervalles von 70°C bis 190°C, Cyclohexan, Methylcyclohexan, Petrolether, Ligroin, Octan, Benzol, Toluol, Xylol; Ester wie Ethylacetat, Isobutylacetat; Amide z.B. Formamid, N-Methylformamid, N,N-Dimethylformamid, N-Methylpyrrolidon; Ketone wie Aceton, Methylethylketon. Auch Gemische der genannten Lösungs- und Verdünnungsmittel kommen in Betracht.

Bevorzugt sind aromatische Kohlenwasserstoffe.

Das Verfahren 2b) wird durchgeführt, indem Verbindungen der Formel (II) und ein Überschuß der Verbindungen der Formel (IV) in einem der angegebenen Verdünnungsmittel zusammengegeben und erhitzt werden. Die Reaktionsdauer beträgt 10 bis 80 Stunden. Nach vollendeter Umsetzung wird abgekühlt, im Vakuum eingeengt, der angefallene Feststoff abfiltriert, gewaschen und getrocknet.

Die Umsetzung erfolgt bei Temperaturen zwischen +50°C und +160°C, vorzugsweise bei Temperaturen zwischen +80°C und +110°C. Es wird bei Normaldruck gearbeitet.

Setzt man bei Verfahren 2c) als Verbindung der Formel (II) (E)-2,3-Dichlor-zimtsäureamidoxim und als Verbindung der Formel (V) Essigsäureanhydrid ein, läßt sich das Verfahren durch folgendes Reaktionsschema wiedergeben:

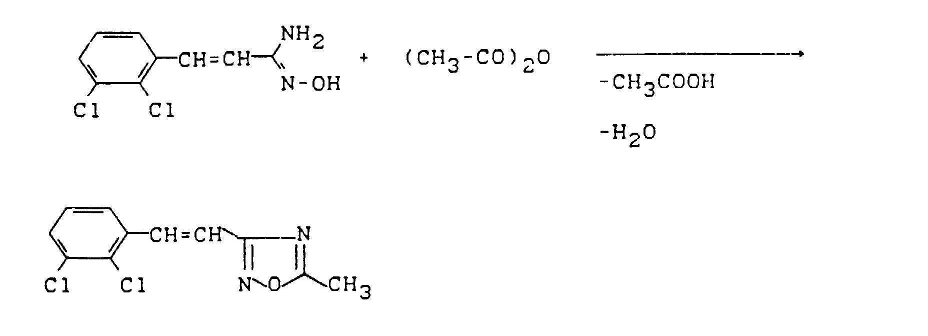

Bevorzugt werden bei Verfahren 2c) die Verbindungen der Formel (II) eingesetzt, bei denen die Reste R, R³ und R⁴ die bei den Verbindungen der Formel (I) genannten bevorzugten und besonders bevorzugten Bedeutungen haben. Die außerdem für die Durchführung des erfindungsgemäßen Verfahrens 2c) als Ausgangsstoffe zu verwendenden Carbonsäureanhydride sind durch die Formel (V) allgemein definiert. Die Carbonsäureanhydride der Formel (V) sind allgemein bekannte Verbindungen der organischen Chemie. Die Reaktionsdurchführung, Aufarbeitung und Isolierung der Reaktionsprodukte erfolgt nach allgemein üblichen Methoden (vgl. auch Herstellungsbeispiele).

Setzt man bei Verfahren 2d) als Verbindung der Formel (II) (E)-3-Chlor-2-fluor-zimtsäureamidoxim und als Verbindung der Formel (VI) Trichloracetylchlorid ein, läßt sich das Verfahren durch folgendes Reaktionsschema beschreiben:

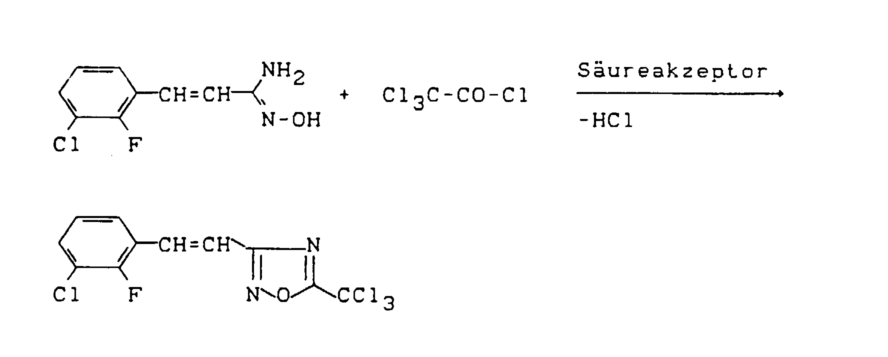

Bevorzugt werden bei Verfahren 2d) die Verbindungen der Formel (II) eingesetzt, bei denen die Reste R, R³ und R⁴ die bei den Verbindungen der Formel (I) genannten bevorzugten und besonders bevorzugten Bedeutungen besitzen. Die außerdem für die Durchführung des erfindungsgemäßen Verfahrens 2d) als Ausgangsstoffe zu verwendenden Carbonsäurechloride sind durch die Formel (VI) allgemein definiert. Die Carbonsäurechloride (VI) sind allgemein bekannte Verbindungen der organischen Chemie oder können nach an sich bekannten Methoden hergestellt werden.

Die Reaktion ist ähnlich der klassischen Tiemann-Acylierungssynthese und wird vorzugsweise unter Verwendung von Verdünnungsmitteln durchgeführt.

Als Verdünnungsmittel zur Durchführung des Verfahrens 2d) finden die bei Verfahren 2b) genannten Halogenkohlenwasserstoffe, insbesondere Chlorkohlenwasserstoffe Verwendung.

Das erfindungsgemäße Verfahren 2d) wird in Gegenwart eines basischen Reaktionshilfsmittels durchgeführt. Als solche können alle geeigneten Säurebindemittel eingesetzt werden wie Amine, insbesondere tertiäre Amine sowie Alkali- und Erdalkaliverbindungen. Beispielhaft seien dafür erwähnt die Hydroxide, Oxide und Carbonate des Lithiums, Natriums, Kaliums, Magnesiums, Calciums und Bariums, ferner weitere basische Verbindungen wie Trimethylamin, Tribenzylamin, Triisopropylamin, Tributylamin, Tribenzylamin, Tricyclohexylamin, Triamylamin, Trihexylamin, N,N-Dimethylanilin, N,N-Dimethyltoluidin, N,N-Dimethyl-p-aminopyridin, N-Methylpyrrolidin, N-Methyl-piperidin, N-Methylimidazol, N-Methylpyrrol, N-Methyl-morpholin, N-Methyl-hexamethylenimin, Pyridin, Chinolin, α-Picolin, β-Picolin, Isochinolin, Pyrimidin, Acridin, N,N.N',N'-Tetra-methylendiamin, N,N,N',N'-Tetraethylendiamin, Chinoxalin, N-Propyl-diisopropylamin, N,N'-Dimethylcyclohexylamin, 2,6-Lutidin, 2,4-Lutidin, Triethylendiamin, Diazabicyclooctan (DABCO), Diazabicyclononen (DBN) oder Diazabicycloundecen (DBU).

Vorzugsweise verwendet man tertiäre Amine, wie beispielsweise Triethylamin oder Pyridin.

Das Verfahren 2d) wird durchgeführt, indem Verbindungen der Formel (II) und ein Überschuß der Verbindungen der Formel (VI) in einem der angegebenen Verdünnungsmittel und in Gegenwart eines basischen Reaktionshilfsmittels zusammengegeben und gegebenenfalls erhitzt werden. Die Reaktionsdauer beträgt 1 bis 6 Stunden. Die Reaktion wird bei Temperaturen zwischen 0°C und +200°C, bevorzugt zwischen +20°C und +150°C durchgeführt. Es wird bei Normaldruck gearbeitet.

Nach vollendeter Umsetzung wird das Reaktionsgemisch filtriert, im Vakuum eingeengt und der verbleibende Rückstand in üblicher Weise durch Umkristallisieren, Vakuumdestillation oder Säulenchromatographie gereinigt.

Bei den erfindungsgemäßen Verfahren 2c), 2d) kann ein O-Acylderivat einer Verbindung der Formel (II) als Zwischenprodukt gebildet werden, das nicht spontan cyclisiert. In solchen Fällen läßt sich die genannte O-Acylverbindung, die als Zwischenprodukt anfällt, beispielsweise durch Erwärmen am Rückfluß zweckdienlich in geeigneten Verdünnungsmitteln, wie aromatischen Kohlenwasserstoffen, cyclisieren.

Die mit Hilfe der erfindungsgemäßen Verfahren 2a), 2b), 2c) oder 2d) erhältlichen substituierten 1,2,4-Oxadiazolderivate der Formel (I) fallen in der Regel als (E)-Isomere an.

Die Wirkstoffe eignen sich bei günstiger Warmblütertoxizität zur Bekämpfung von pathogenen Endoparasiten die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla und Waschbär, Vögel wie z.B. Hühner, Gänse, Puten und Enten, Süß- und Salzwasserfische wie z.B. Forellen, Karpfen und Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspension zur oralen oder dermalen Anwendung sowie zur Injektion; Halbfeste Zubereitungen;
Formulierungen bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmono-methylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B.DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/ Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-manoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien und Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff Beispiel Nr. | Dosis effectiva in mg/kg |
|---|---|
| 2 | 10 |
| 3 | 10 |
| 39 | 10 |
| 40 | 10 |
| 47 | 10 |

### Beispiel B

### In vivo Nematodentest

### Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzeiren (Dosis effectiva).

Geprüfte Wirkstoffe und wirksame Dosierungen (Dosis effectiva) sind aus der nachfolgenden Tabelle ersichtlich:

| Wirkstoff Beispiel Nr. | Dosis effectiva in mg/kg |
|---|---|
| 3 | 10 |
| 39 | 10 |
| 47 | 10 |

### Herstellungsbeispiele

### Beispiel 1

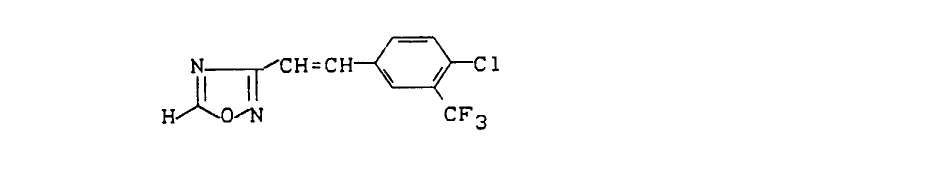

5,2 g (0,02 Mol) (E)-4-Chlor-3-trifluormethyl-zimtsäureamidoxim werden in 50 ml Orthoameisensäuretriethylester vorgelegt und bei Raumtemperatur 2 Tropfen Bortrifluorid-etherat zugegeben. Dann wird 2 Stunden bei Rückflußtemperatur gerührt und der gesamte Ansatz im Vakuum eingeengt. Der Rückstand wird in 100 ml Methylenchlorid aufgenommen und nacheinander mit 100 ml 2N-Salzsäure, gesättigter Natriumcarbonatlösung und Wasser gewaschen. Die organische Phase wird über Natriumsulfat getrocknet und anschließend das Lösungsmittel abdestilliert. Man erhält 3,9 g (71,0 % der Theorie) (E)-3-(4-Chlor-3-trifluormethyl-styryl)-1,2,4-oxadiazol.
Fp.: 85 bis 87°C
¹H-NMR (CDCl₃, δ): 7,17; 7,71 (2d, =CH; J_{H,H} = 16,3 Hz; E-Form); 7,24-8,70 (3m, arom.); 9,68 (s, =CH) ppm

Analog können die in der nachstehenden Tabelle 1 aufgeführten Verbindungen der Formel (Ia, R¹ = H) hergestellt werden.

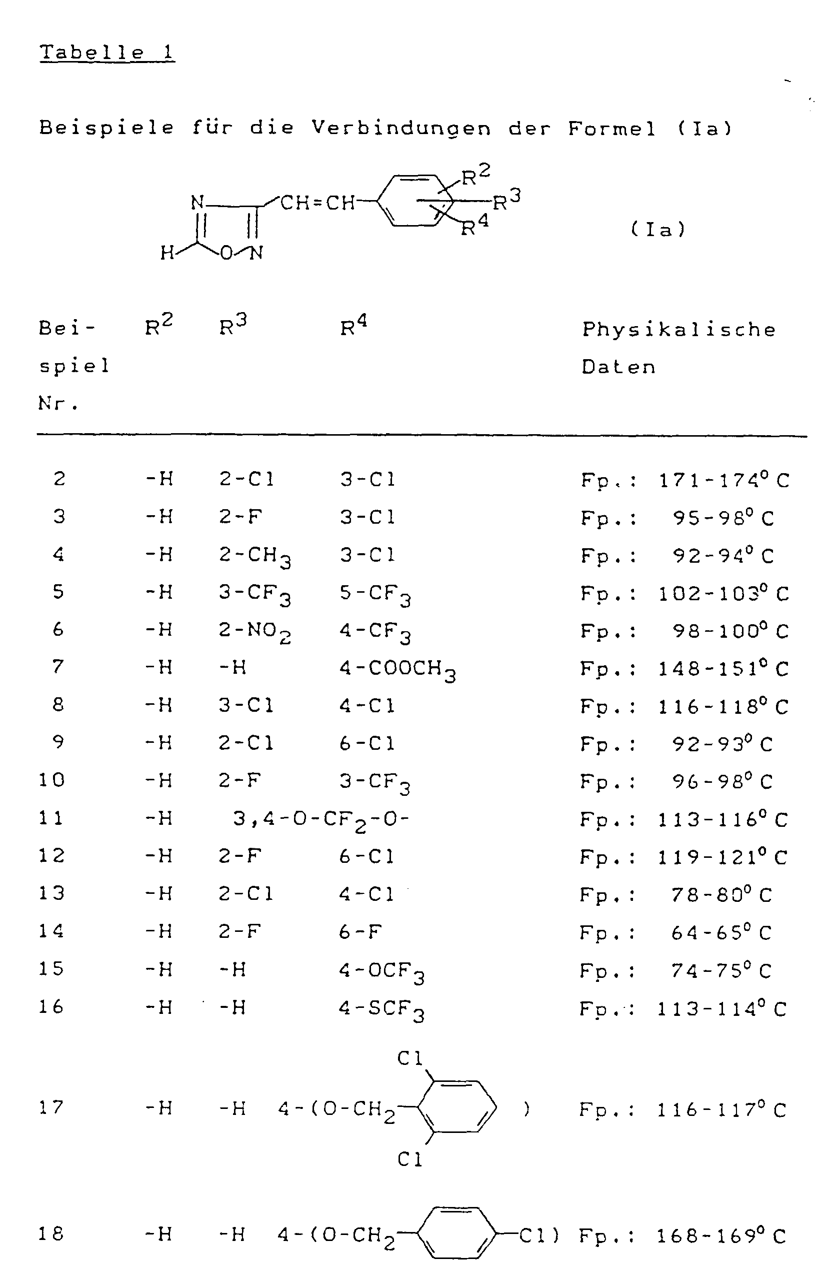

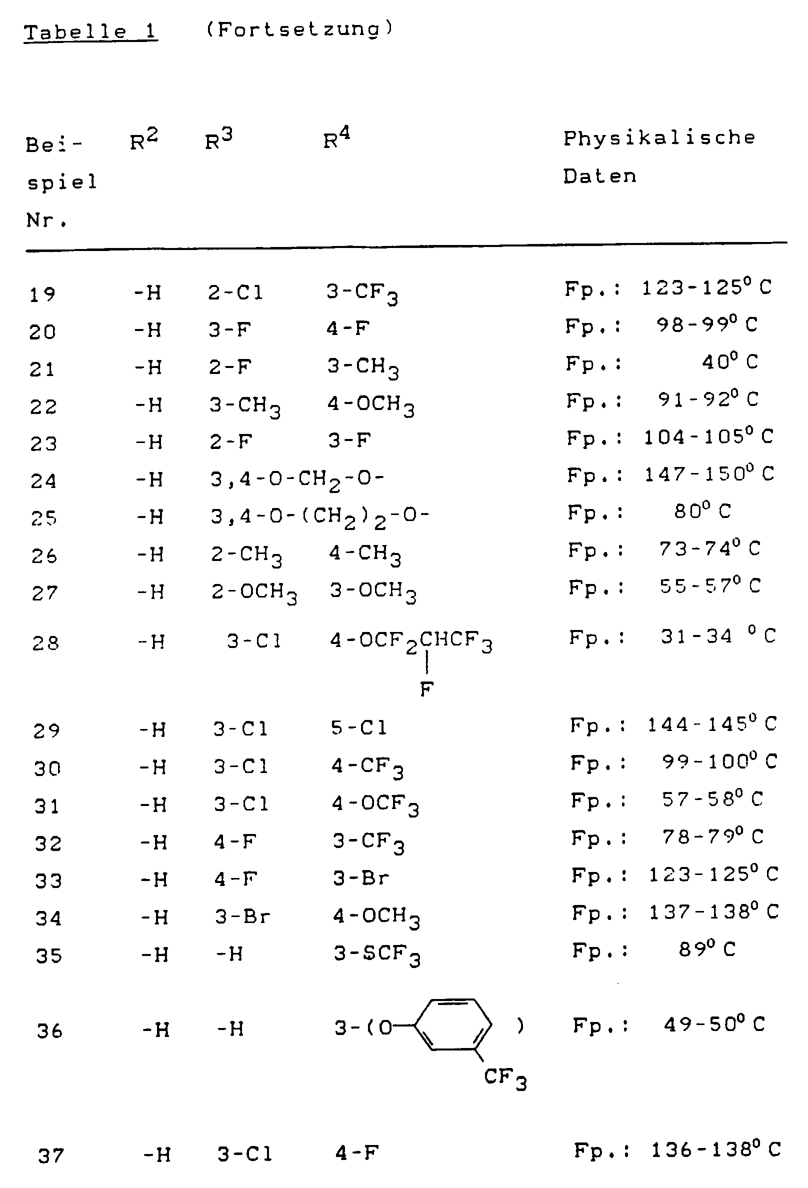

### Beispiel 38

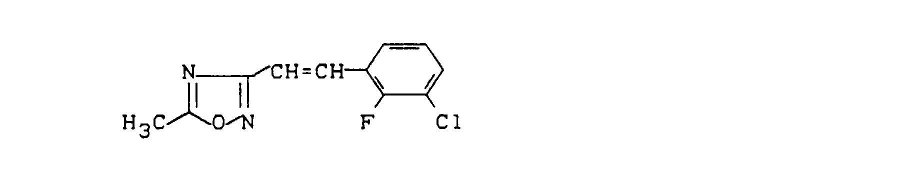

6,4 g (0,03 Mol) (E)-3-Chlor-2-fluor-zimtsäureamidoxim werden in 50 ml Essigsäureanhydrid vorgelegt und 2 Stunden bei Rückflußtemperatur gerührt. Anschließend wird der gesamte Ansatz im Vakuum eingeengt, der Rückstand mit Natriumcarbonatlösung verrührt und mit Methylenchlorid extrahiert. Die organische Phase wird über Natriumsulfat getrocknet und das Lösungsmittel abdestilliert. Man erhält 5,1 g (75,6 % der Theorie) (E)-3-(3-Chlor-2-fluor-styryl)-5-methyl-1,2,4-oxadiazol.
Fp.: 95 bis 98°C
¹H-NMR (CDCl₃, δ): 2,60 (s, -CH₃); 7,16; 7,76 (2d, =CH; J_{H,H} = 16,5 Hz; E-Form); 7,09-7,51 (m, arom.) ppm

Analog können die in der nachstehenden Tabelle 2 aufgeführten Verbindungen der Formel (Ib, R¹ = -Me) hergestellt werden.

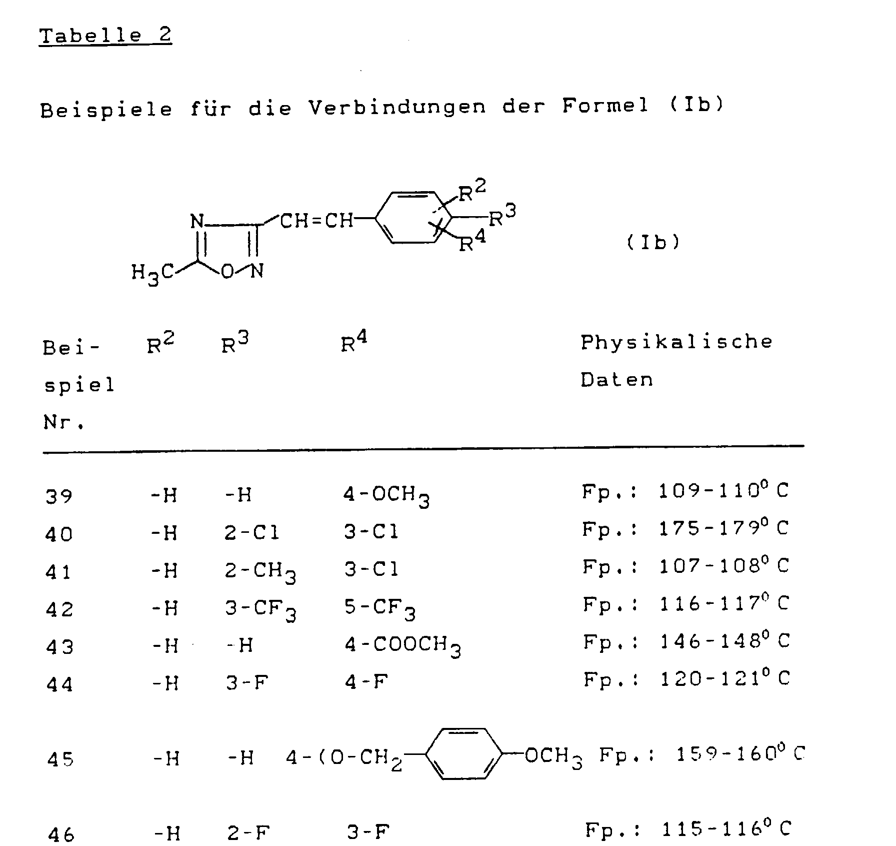

### Beispiel 47

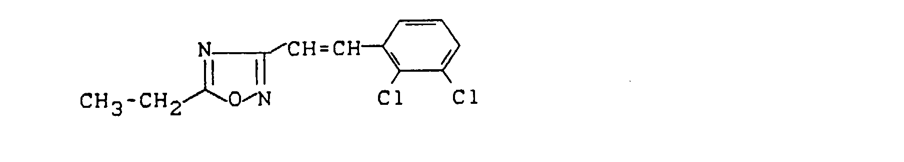

7,0 g (0,03 Mol) (E)-2,3-Dichlor-zimtsäureamidoxim werden in 7 ml Propionsäureanhydrid vorgelegt und 2 Stunden bei Rückflußtemperatur gerührt. Anschließend wird der gesamte Ansatz im Vakuum eingeengt, der Rückstand mit Natriumcarbonatlösung verrührt und mit Methylenchlorid extrahiert. Die organische Phase wird über Natriumsulfat getrocknet und das Lösungsmittel abdestilliert. Man erhält 4,7 g (58,2 % der Theorie) (E)-3-(2,3-Dichlor-styryl)-5-ethyl-1,2,4-oxadiazol.
Fp.: 107 bis 109°C
¹H-NMR (CDCl₃, δ): 1,44 (t, -CH₃; J_{H,H} = 7,6 Hz); 2,95 (q, -CH₂-; J_{H,H} = 7,6 Hz); 7,02; 8,07 (2d, =CH; J_{H,H} = 16,0 Hz); 7,21-7,59 (3m, arom.) ppm

Analog können die in der nachstehenden Tabelle 3 aufgeführten Verbindungen der Formel (Ic, R¹ = -Et) hergestellt werden.

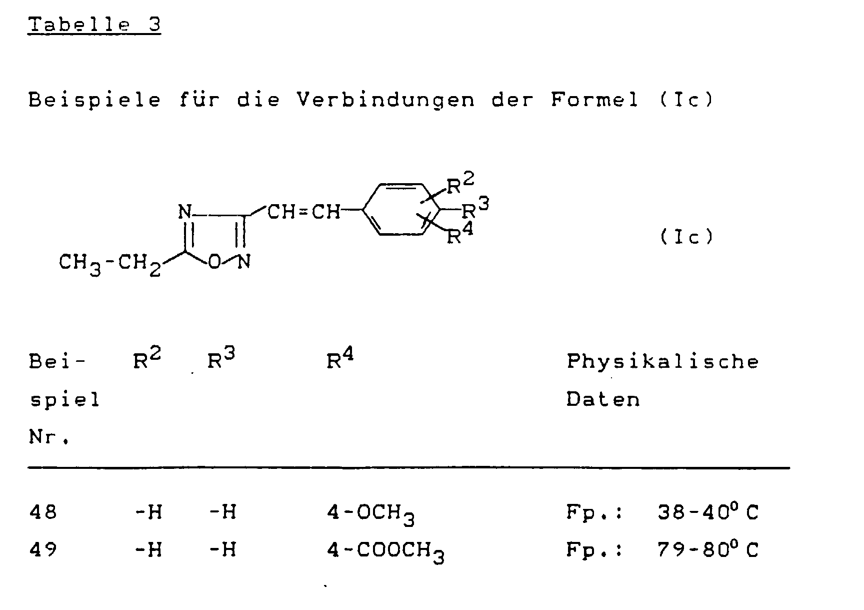

### Beispiel 50

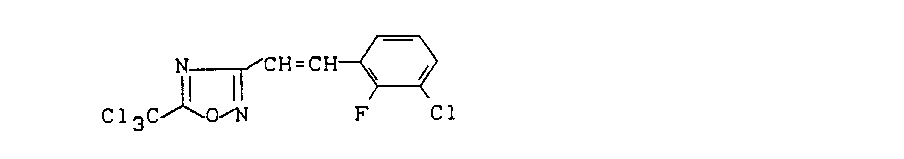

Zu einer Suspension aus 6,4 g (0,03 Mol) (E)-3-Chlor-2-fluor-zimtsäureamidoxim in 90 ml Chloroform und 6 ml (0,07 Mol) trockenem Pyridin werden 12,7 g (0,07 Mol) Trichloracetylchlorid getropft. Dabei tritt eine leichte Wärmeentwicklung auf. Anschließend wird 1 Stunde nachgerührt, das dabei abscheidende Pyridinhydrochlorid abfiltriert und mit Chloroform gewaschen. Das Filtrat wird im Vakuum eingeengt und der zurückbleibende Feststoff umkristallisiert. Man erhält 5,0 g (48,7 % der Theorie) (E)-3-(3-Chlor-2-fluor-styryl)-5-trichlormethyl-1,2,4-oxadiazol.
Fp.: 37 bis 38°C
¹H-NMR (CDCl₃, δ): 7,12-7,52 (m, arom. + =CH); 7,87 (d, = CH; J_{H,H} = 16,0 Hz) ppm

Analog können die in der nachstehenden Tabelle 4 aufgeführten Verbindungen der Formel (Id, R¹ = -CCl₃) hergestellt werden.

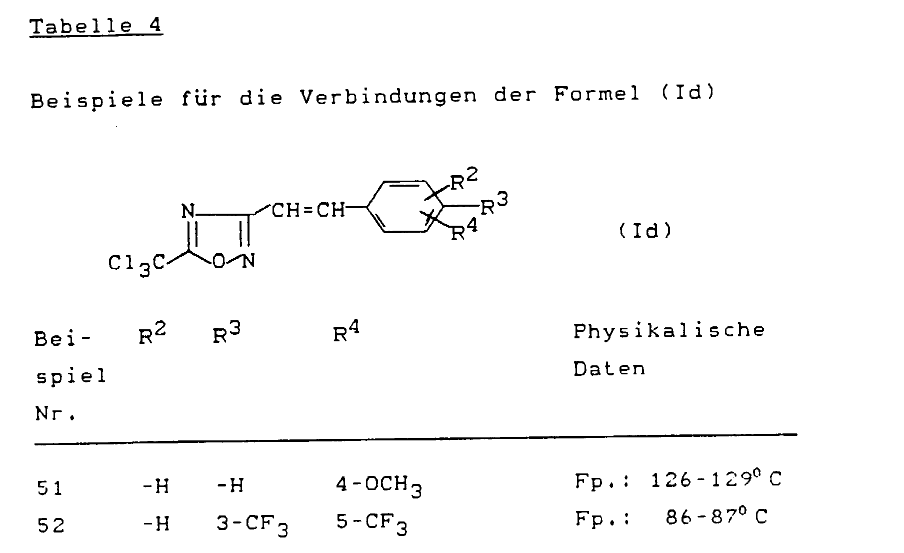

### Beispiel 53

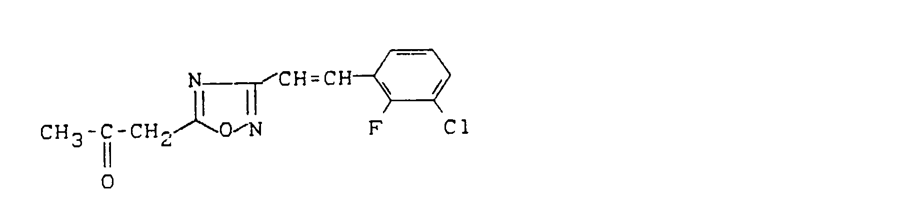

4,3 g (0,02 Mol) (E)-3-Chlor-2-fluor-zimtsäureamidoxim wurden in 85 ml Toluen vorgelegt, mit 16,9 g (0,13 Mol) Acetessigsäureethylester versetzt und bis zum vollständigen Umsatz (45 Stunden) bei Rückflußtemperatur gerührt. Danach wird der gesamte Ansatz im Vakuum eingeengt und der verbleibende Rückstand umkristallisiert.

Man erhält 4,3 g (76,6 % der Theorie) (E)-3-(3-Chlor-2-fluor-styryl)-5-(2-oxopropyl)-1,2,4-oxadiazol als Keto-Enol-Tautomerengemisch (81:19).
Fp.: 75 bis 77°C
¹H-NMR (CDCl₃, δ): 2,15; 2,36 (2s, -CH₃); 4,09 (s, -CH₂-); 5,56 (s, =CH); 7,18; 7,7; 7,78 (3d, =CH, J_{H,H} = 16,3 Hz; E-Form); 7,10-7,51 (m, arom. + =CH); 11,40 (s, -OH) ppm

Analog können die in der nachstehenden Tabelle 5 aufgeführten Verbindungen der Formel (Ie, R¹ = -CH₂COCH₃) hergestellt werden.

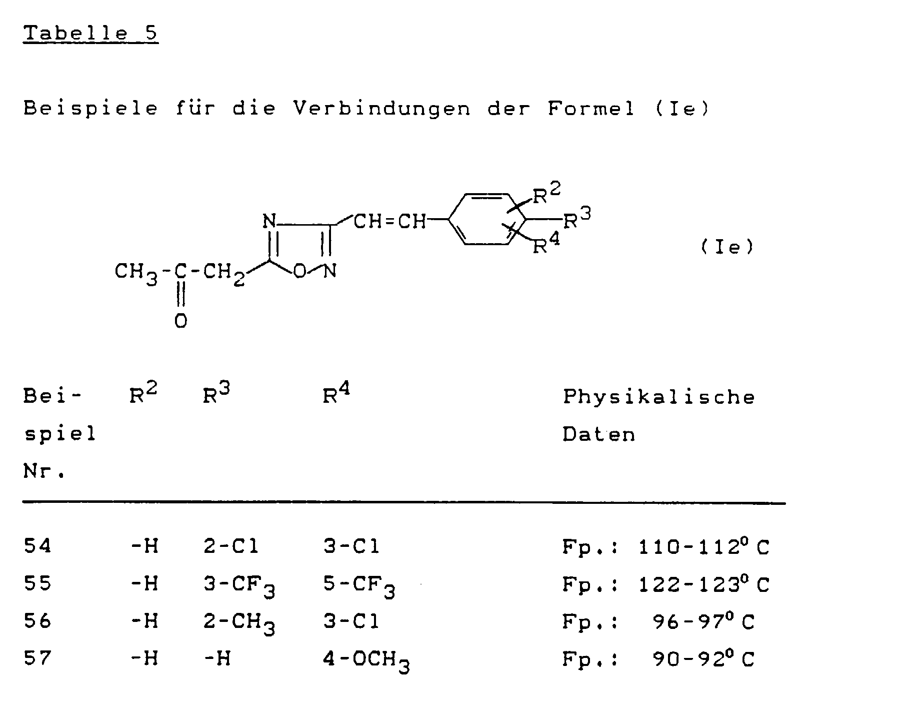

### Ausgangsstoffe der Formel (II)

### Beispiel (II-1)

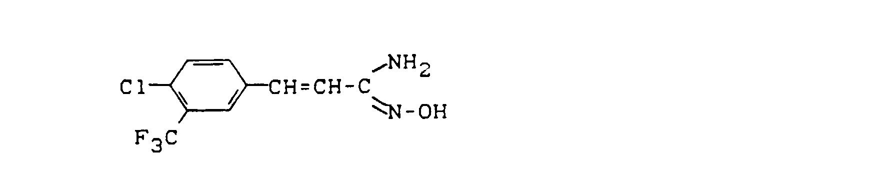

Zu einer Lösung aus 7,2 g (0,10 Mol) Hydroxylaminhydrochlorid, 7,3 g (0,10 Mol) Natriumcarbonat in 100 ml Wasser, 100 ml Ethanol werden 12,1 g (0,05 Mol) 4-Chlor-3-trifluormethyl-zimtsäurenitril gegeben. Die Mischung wird bis zum vollständigen Umsatz (24 Stunden) auf Rückflußtemperatur erhitzt und anschließend wird der gesamte Ansatz in 250 ml Wasser eingerührt. Der dabei abscheidende Feststoff wird abgesaugt, mit wenig Wasser gewaschen und getrocknet. Man erhält 5,3 g (39,3 % der Theorie) (E)-4-Chlor-3-trifluormethyl-zimtsäureamidoxim.
Fp.: 135 bis 137°C
¹H-NMR (CDCl₃, δ): 5,64 (br s, -NH₂); 6,60; 7,15 (2d, =CH; J_{H,H} = 16,5 Hz; E-Form); 7,68-10,00 (m, arom.); 11,89 (s, -OH) ppm

Analog können die in der nachstehenden Tabelle 6 aufgeführten Verbindungen der Formel (II) hergestellt werden.

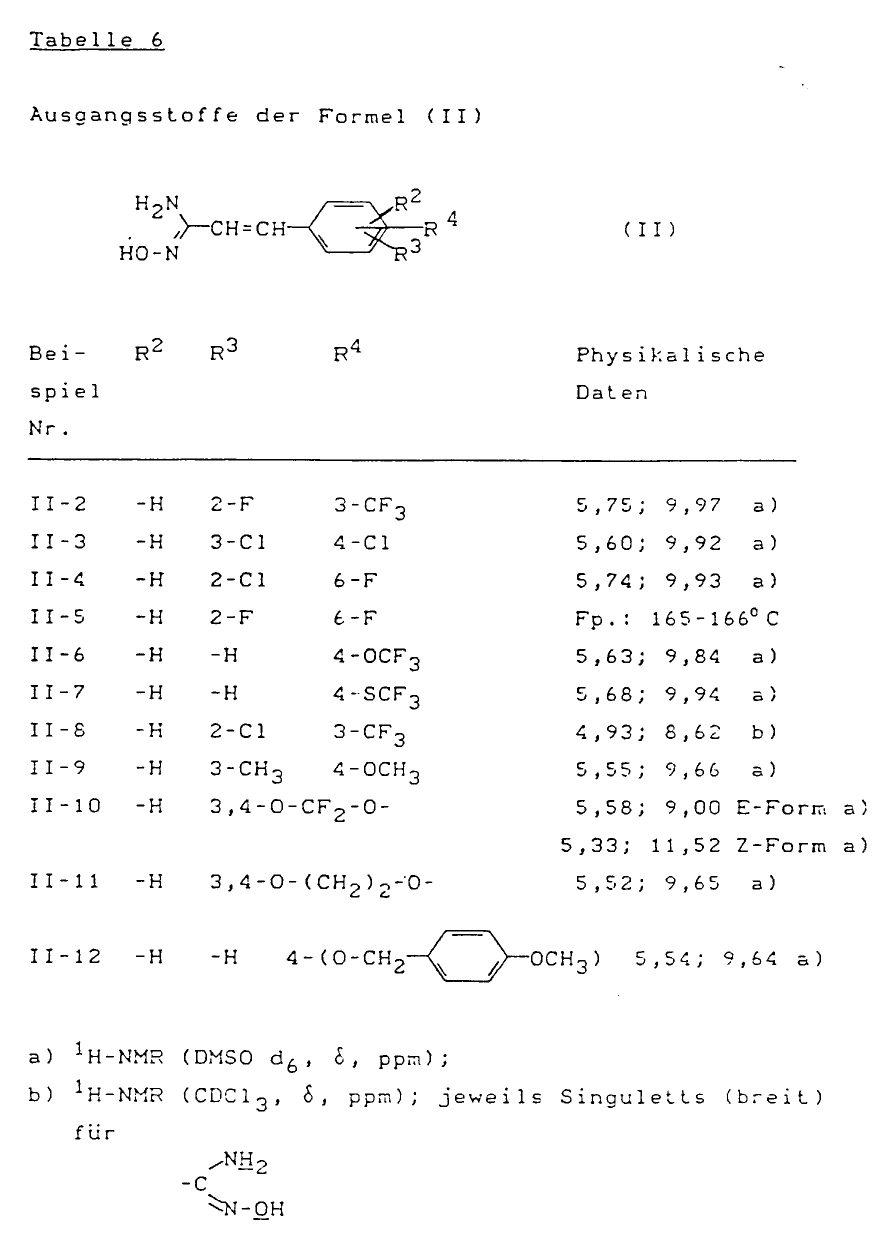

Beispiel zur Herstellung der als Vorstufe eingesetzten substituierten Zimtsäurenitrile (vgl. z.B. Foucaud et al. Synthesis (1979), S. 884 bis 885):

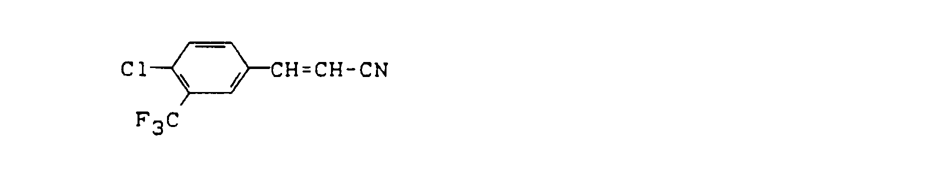

Zu einer Suspension aus 6,7 g (0,12 Mol) pulverisiertem Kaliumhydroxid in 185 ml Tetrahydrofuran wird eine Lösung aus 10,6 g (0,06 Mol) Cyanmethylphosphonsäurediethylester und 12,5 g (0,06 Mol) 4-Chlor-3-trifluor-methyl-benzaldehyd in 65 ml Tetrahydrofuran getropft. Dabei tritt eine leichte Wärmeentwicklung auf. Anschlie-ßend werden 20 Minuten bei Raumtemperatur nachgerührt, der dabei abscheidende Feststoff wird abfiltriert und mit Tetrahydrofuran gewaschen. Das Filtrat wird im Vakuum eingeengt und der verbleibende Rückstand getrocknet. Man erhält 12,1 g (87,0 % der Theorie) 4-Chlor-3-trifluormethyl-zimtsäurenitril als E/Z-Isomerengemisch.
Fp.: 89-92° C
¹H-NMR (CDCl₃, δ): 5,60; 7,13 (2d, =CH, J_{H,H} = 12 Hz; Z-Form); 5,96; 7,38 (2d, =CH, J_{H,H} = 16,5 Hz; E-Form); 7,57-8,07 (m, arom.) ppm

Analog können die in der nachstehenden Tabelle 7 aufgeführten Verbindungen hergestellt werden.

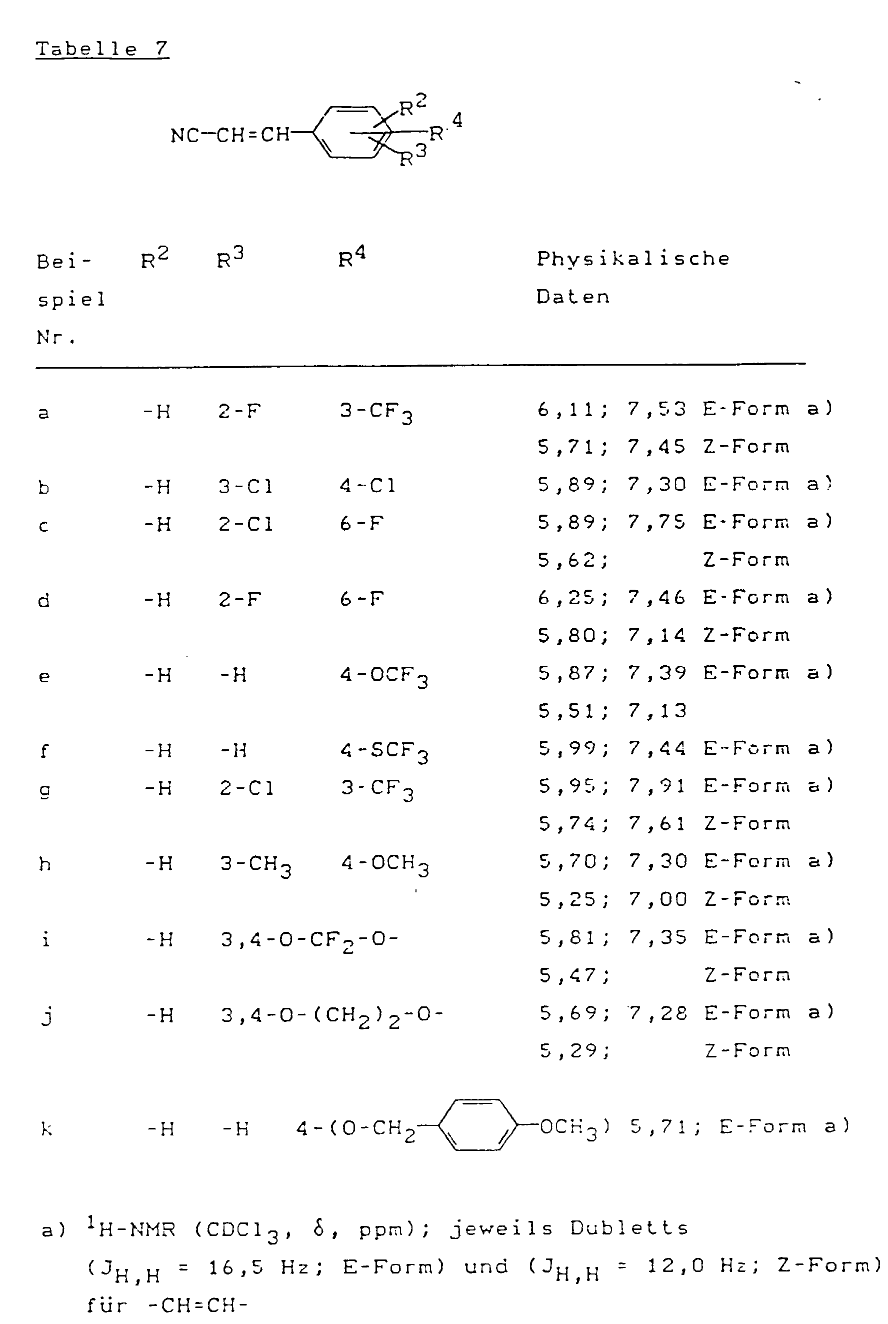

## Patentansprüche

1. Substituierte 1,2,4-Oxadiazolderivate der allgemeinen Formel (I) und ihre Stereoisomere 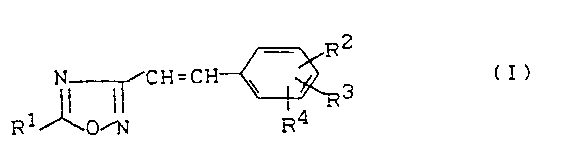 in welcher
R¹ für Wasserstoff, C₁₋₆-Alkyl, das gegebenenfalls substituiert ist durch 1 bis 6 Halogenatome, C₃₋₆-Cycloalkyl, C₁₋₆-Alkoxy, Hydroxy, gegebenenfalls substituiertes Phenyl, C₁₋₄-Alkyl-carbonyl, C₁₋₆-Alkoxycarbonyl sowie für C₃₋₆-Cycloalkyl steht,
R für Wasserstoff, Halogen, CN, NO₂, C₁₋₆-Alkyl, 1 bis 6 Halogen-C₁₋₆-Alkyl, C₁₋₆-Alkoxy oder C₁₋₆-Thioalkyl steht,
R³ für einen der bei R angegebenen Reste steht,
R⁴ für SH, 1 bis 5 Halogen-C₁₋₆-alkoxy, 1 bis 6 Halogen-C₁₋₄-alkylthio, 1 bis 6 Halogen-C₁₋₆-alkylsulfinyl, 1 bis 5 Halogen-C₁₋₆-alkylsulfonyl, gegebenenfalls substituiertes Phenoxy, gegebenenfalls substituiertes Phenylmercapto, gegebenenfalls substituiertes Phenyl-C₁₋₆-alkoxy, gegebenenfalls substituiertes Phenyl-C₁₋₆-alkylthio, C₁₋₂-Alkylendioxy, 1 bis 4 Halogen-C₁₋₂-alkylendioxy, C₁₋₆-Alkylamino, Di-C₁₋₄-alkylamino, Acetylamino, Benzolsulfonylamino, -CONH₂, -CONH(C₁₋₄-Alkyl), C₁₋₄-Alkoxycarbonyl, C₁₋₄-Alkylcarbonyl, C₁₋₄-Alkylcarbonyloxy, C₁₋₄-Alkoxy-C₁₋₄-alkyl, C₁₋₄-Alkyl-C₁₋₄-alkoxy und Hydroxy-C₁₋₄-alkoxy steht,
sowie für den Fall, daß mindestens einer der Reste R und R³ eine andere Bedeutung als Wasserstoff hat, zusätzlich für Halogen, C₁₋₄-Alkyl, 1 bis 6 Halogen-C₁₋₄-alkyl,C₁₋₄-Alkoxy und C₁₋₄-Alkylthio, OH steht, sowie für den Fall, daß R¹ für gegebenenfalls substituiertes C₁₋₆-Alkyl steht, zusätzlich für C₁₋₄-Alkoxy und NH₂ steht,
wobei die Substituenten der gegebenenfalls substituierten Reste
Halogen, C₁-C₄-Alkyl, C₁-C₄-Halogenalkyl, C₁-C₄-Alkoxy C₁-C₄-Halogenalkoxy, C₁-C₄-Halogenalkylthio, C₁-C₄-Halogenalkylsulfinyl, C₁-C₄-Halogenalkylsulfonyl, Methylendioxy oder Ethylendioxy, die gegebenenfalls durch Fluor oder Chlor substituiert sind, Amino, C₁-C₄-Alkyl- und Dialkylamino, Acetylamino sein können.

2. Verfahren zur Herstellung der substituierten 1,2,4-Oxadiazolderivate der Formel (I) und deren Stereoisomere, 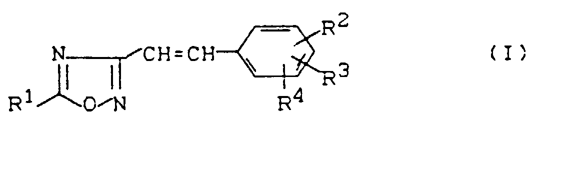 in welcher
R ¹ für Wasserstoff, C₁₋₆-Alkyl, das gegebenenfalls substituiert ist durch 1 bis 6 Halogenatome, C₃₋₆-Cycloalkyl, C₁₋₆-Alkoxy, Hydroxy, gegebenenfalls substituiertes Phenyl, C₁₋₄-Alkyl-carbonyl, C₁₋₆-Alkoxycarbonyl sowie für C₃₋₆-Cycloalkyl steht,
R für Wasserstoff, Halogen, CN, NO₂, C₁₋₆-Alkyl, 1 bis 6 Halogen-C₁₋₆-Alkyl, C₁₋₆-Alkoxy oder C₁₋₆-Thioalkyl steht,
R³ für einen der bei R angegebenen Reste steht,
R⁴ für SH, 1 bis 5 Halogen-C₁₋₆-alkoxy, 1 bis 6 Halogen-C₁₋₄-alkylthio, 1 bis 6 Halogen-C₁₋₆-alkylsulfinyl, 1 bis 5 Halogen-C₁₋₆-alkylsulfonyl, gegebenenfalls substituiertes Phenoxy, gegebenenfalls substituiertes Phenylmercapto, gegebenenfalls substituiertes Phenyl-C₁₋₆-alkoxy, gegebenenfalls substituiertes Phenyl-C₁₋₆-alkylthio, C₁₋₃-Alkylendioxy, 1 bis 4 Halogen-C₁₋₂-alkylendioxy, C₁₋₆-Alkylamino, Di-C₁₋₄-alkylamino, Acetylamino, Benzolsulfonylamino, -CONH₂, -CONH(C₁₋₄-Alkyl), C₁₋₄-Alkoxycarbonyl, C₁₋₄-Alkylcarbonyl, C₁₋₄-Alkylcarbonyloxy, C₁₋₄-Alkoxy-C₁₋₄-alkyl, C₁₋₄-Alkyl-C₁₋₄-alkoxy und Hydroxy-C₁₋₄-alkoxy steht,
sowie für den Fall, daß mindestens einer der Reste R und R³ eine andere Bedeutung als Wasserstoff hat, zusätzlich für Halogen, C₁₋₄-Alkyl, 1 bis 6 Halogen-C₁₋₄-alkyl,C₁₋₄-Alkoxy und C₁₋₄-Alkylthio OH steht, sowie für den Fall, daß R¹ für gegebenenfalls substituiertes C₁₋₆-Alkyl steht, zusätzlich für C₁₋₄-Alkoxy und NH₂ steht,
wobei die Substituenten der gegebenenfalls substituierten Reste
Halogen, C₁-C₄-Alkyl, C₁-C₄-Halogenalkyl, C₁-C₄-Alkoxy, C₁-C₄-Halogenalkoxy, C₁-C₄-Halogenalkylthio, C₁-C₄-Halogenalkylsulfinyl, C₁-C₄-Halogenalkylsulfonyl, Methylendioxy oder Ethylendioxy, die gegebenenfalls durch Fluor oder Chlor substituiert sind, Amino, C₁-C₄-Alkyl- und Dialkylamino, Acetylamino sein können.
dadurch gekennzeichnet, daß man ein Amidoximderivat der Formel (II) und Stereoisomere davon 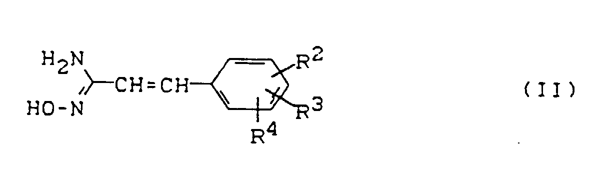 in welcher
R, R³ und R⁴ die oben angegebenen Bedeutungen besitzen,
a) mit einem Carbonsäureorthoester der Formel (III)
R¹-C(O-A)₃ (III)
in welcher
R¹ die oben angegebene Bedeutung besitzt und
A für Alkyl, insbesondere für Methyl oder Ethyl steht,
umsetzt oder
b) mit einem Carbonsäureester der Formel (IV) 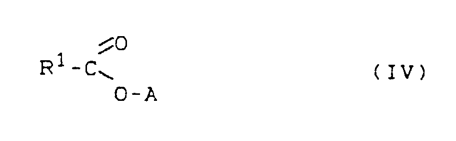 in welcher
R¹ und A die oben angegebene Bedeutung haben umsetzt oder
c) mit Carbonsäureanhydriden der Formel (V)
(R¹-CO)₂O (V)
in welcher
R¹ die oben angegebene Bedeutung besitzt
umsetzt oder
d) mit einem Carbonsäurehalogenid der Formel (VI) 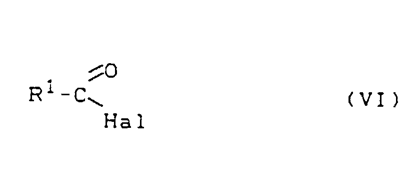 in welcher
R¹ die oben angegebene Bedeutung hat und
Hal für Halogen, steht,
in Gegenwart eines Verdünnungsmittels und in Gegenwart eines Reaktionshilfsmittels umsetzt.

3. Endoparasitizide Mittel, gekennzeichnet durch einen Gehalt an mindestens einem 1,2,4-Oxadiazolderivat der Formel (I) gemäß Anspruch 1.

4. Verwendung von 1,2,4-Oxadiazolderivaten der Formel (I) gemäß Anspruch 1 zur Herstellung von endoparasitiziden Mitteln.

## Claims

1. Substituted 1,2,4-oxadiazole derivatives of the general formula (I) and their stereoisomers 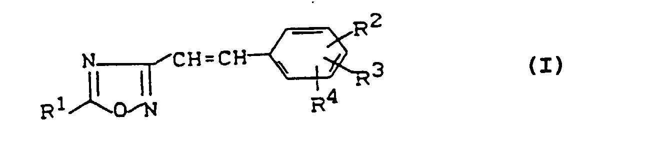 in which formula
R¹ represents hydrogen, C₁₋₆-alkyl which is optionally substituted by 1 to 6 halogen atoms, C₃₋₆-cycloalkyl, C₁₋₆-alkoxy, hydroxyl, optionally substituted phenol, C₁₋₄-alkylcarbonyl, C₁₋₆-alkoxycarbonyl, and C₃₋₆-cycloalkyl,
R represents hydrogen, halogen, CN, NO₂, C₁₋₆-alkyl, 1 to 6 halogeno-C₁₋₆-alkyl, C₁₋₆-alkoxy or C₁₋₆-thioalkyl,
R³ represents one of the radicals given for R,
R⁴ represents SH, 1 to 5 halogeno-C₁₋₆-alkoxy, 1 to 6 halogeno-C₁₋₄-alkylthio, 1 to 6 halogeno-C₁₋₆-alkylsulphinyl, 1 to 5 halogeno-C₁₋₆-alkylsulphonyl, optionally substituted phenoxy, optionally substituted phenylmercapto, optionally substituted phenyl-C₁₋₆-alkoxy, optionally substituted phenyl-C₁₋₆-alkylthio, C₁₋₂-alkylenedioxy, 1 to 4 halogeno-C₁₋₂-alkylenedioxy, C₁₋₆-alkylamino, di-C₁₋₄-alkylamino, acetylamino, benzenesulphonylamino, -CONH₂, -CONH(C₁₋₄-alkyl), C₁₋₄-alkoxycarbonyl, C₁₋₄-alkylcarbonyl, C₁₋₄-alkylcarbonyloxy, C₁₋₄-alkoxy-C₁₋₄-alkyl, C₁₋₄-alkyl-C₁₋₄-alkoxy and hydroxy-C₁₋₄-alkoxy,
and, in the event that at least one of the radicals R and R³ has a meaning other than hydrogen, additionally represents halogen, C₁₋₄-alkyl, 1 to 6 halogeno-C₁₋₄-alkyl, C₁₋₄-alkoxy and C₁₋₄-alkylthio, OH, and in the event that R¹ represents optionally substituted C₁₋₆-alkyl, additionally represents C₁₋₄-alkoxy and NH₂,
it being possible for the substituents of the optionally substituted radicals to be halogen, C₁-C₄-alkyl, C₁-C₄-halogenoalkyl, C₁-C₄-alkoxy, C₁-C₄-halogenoalkoxy, C₁-C₄-halogenoalkylthio, C₁-C₄-halogenoalkylsulphinyl, C₁-C₄-halogenoalkylsulphonyl, methylenedioxy or ethylenedioxy, optionally substituted by fluorine or chlorine, or amino, C₁-C₄-alkyl- and dialkylamino and acetylamino.

2. Processes for the preparation of the substituted 1,2,4-oxadiazole derivatives of the formula (I) and their stereoisomers 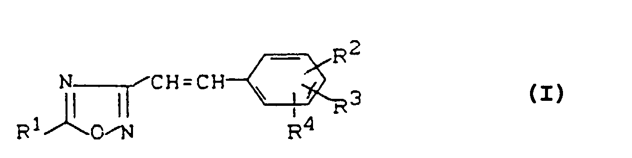 in which formula
R¹ represents hydrogen, C₁₋₆-alkyl which is optionally substituted by 1 to 6 halogen atoms, C₃₋₆-cycloalkyl, C₁₋₆-alkoxy, hydroxyl, optionally substituted phenyl, C₁₋₄-alkylcarbonyl, C₁₋₆-alkoxycarbonyl, and C₃₋₆-cycloalkyl,
R represents hydrogen, halogen, CN, NO₂, C₁₋₆-alkyl, 1 to 6 halogeno-C₁₋₆-alkyl, C₁₋₆-alkoxy or C₁₋₆-thioalkyl,
R³ represents one of the radicals given for R,
R⁴ represent SH, 1 to 5 halogeno-C₁₋₆-alkoxy, 1 to 6 halogeno-C₁₋₄-alkylthio, 1 to 6 halogeno-C₁₋₆-alkylsulphinyl, 1 to 5 halogeno-C₁₋₆-alkyl-sulphonyl, optionally substituted phenoxy, optionally substituted phenylmercapto, optionally substituted phenyl-C₁₋₆-alkoxy, optionally substituted phenyl-C₁₋₆-alkylthio, C₁₋₂-alkylenedioxy, 1 to 4 halogeno-C₁₋₂-alkylenedioxy, C₁₋₆-alkylamino, di-C₁₋₄-alkylamino, acetylamino, benzenesulphonylamino, -CONH₂, -CONH(C₁₋₄-alkyl), C₁₋₄-alkoxycarbonyl, C₁₋₄-alkylcarbonyl, C₁₋₄-alkylcarbonyloxy, C₁₋₄-alkoxy-C₁₋₄-alkyl, C₁₋₄-alkyl-C₁₋₄-alkoxy and hydroxy-C₁₋₄-alkoxy,
and, in the event that at least one of the radicals R and R³ has a meaning other than hydrogen, additionally represents halogen, C₁₋₄-alkyl, 1 to 6 halogeno-C₁₋₄-alkyl, C₁₋₄-alkoxy and C₁₋₄-alkylthio, OH, and in the event that R¹ represents optionally substituted C₁₋₆-alkyl, additionally represents C₁₋₄-alkoxy and NH₂,
it being possible for the substituents of the optionally substituted radicals to be halogen, C₁-C₄-alkyl, C₁-C₄-halogenoalkyl, C₁-C₄-alkoxy, C₁-C₄-halogenoalkoxy, C₁-C₄-halogenoalkylthio, C₁-C₄-halogenoalkylsulphinyl, C₁-C₄-halogenoalkylsulphonyl, methylenedioxy or ethylenedioxy, optionally substituted by fluorine or chlorine, or amino, C₁-C₄-alkyl- and dialkylamino,
characterised in that an amide oxime derivative of the formula (II) and stereoisomers thereof 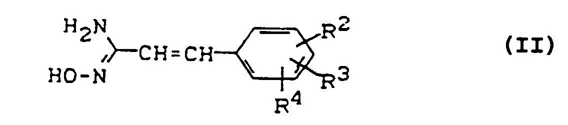 in which formula
R, R³ and R⁴ have the abovementioned meanings,
a) are reacted with a carboxylic acid orthoester of the formula (III)
R¹-C(O-A)₃ (III)
in which
R¹ has the abovementioned meaning and
A represents alkyl, in particular methyl or ethyl,
or
b) are reacted with a carboxylic acid ester of the formula (IV) 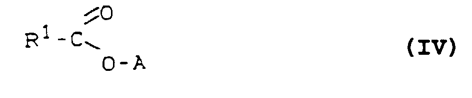 in which
R¹ and A have the abovementioned meaning
or
c) are reacted with carboxylic anhydrides of the formula (V)
(R¹-CO)₂O (V)
in which
R¹ has the abovementioned meaning
or
d) are reacted with a carboxylic acid halide of the formula (VI) 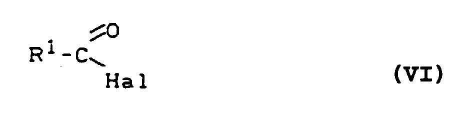 in which
R¹ has the abovementioned meaning and
Hal represents halogen, in the presence of a diluent and in the presence of a reaction auxiliary.

3. Endoparasiticidal agents, characterised in that they contain at least one 1,2,4-oxadiazole derivative of the formula (I) according to Claim 1.

4. Use of 1,2,4-oxadiazole derivatives of the formula (I) according to Claim 1 for the preparation of endoparasiticidal agents.

## Revendications

1. Dérivés substitués de 1,2,4-oxadiazoles de formule générale (I) et leurs stéréo-isomères, 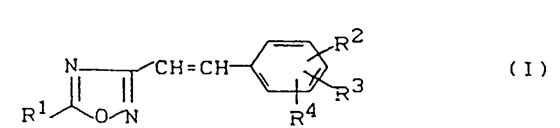 formule dans laquelle
R¹ représente l'hydrogène, un groupe alkyle en C₁ à C₆ qui est substitué le cas échéant par 1 à 6 atomes d'halogènes, cycloalkyle en C₃ à C₆, alkoxy en C₁ à C₆, hydroxy, phényle éventuellement substitué, (alkyle en C₁ à C₄)carbonyle, (alkoxy en C₁ à C₆)carbonyle ainsi qu'un groupe cycloalkyle en C₃ à C₆,
R représente de l'hydrogène, un halogène, un groupe CN, NO₂, alkyle en C₁ à C₆, alkyle en C₁ à C₆ portant 1 à 6 atomes d'halogènes, alkoxy en C₁ à C₆ ou thioalkyle en C₁ à C₆,
R³ représente l'un des restes indiqués pour R,
R⁴ est un groupe SH, un groupe alkoxy en C₁ à C₆ portant 1 à 5 halogènes, un groupe alkylthio en C₁ à C₄ portant 1 à 6 halogènes, un groupe alkylsulfinyle en C₁ à C₆ portant 1 à 6 halogènes, un groupe alkylsulfonyle en C₁ à C₆ portant 1 à 5 halogènes, un groupe phénoxy éventuellement substitué, phénylmercapto éventuellement substitué, phényl-(alkoxy en C₁ à C₆) éventuellement substitué, phényl-(alkylthio en C₁ à C₆) éventuellement substitué, alkylènedioxy en C₁ ou C₂, alkylènedioxy en C₁ ou C₂ portant 1 à 4 halogènes, alkylamino en C₁ à C₆, di(alkyle en C₁ à C₄)amino, acétylamino, benzènesulfonylamino, -CONH₂, -CONH(alkyle en C₁ à C₄), (alkoxy en C₁ à C₄)-carbonyle, (alkyle en C₁ à C₄)-carbonyle, (alkyle en C₁ à C₄)-carbonyloxy, (alkoxy en C₁ à C₄)-(alkyle en C₁ à C₄), (alkyle en C₁ à C₄)-(alkoxy en C₁ à C₄) et hydroxy(alkoxy en C₁ à C₄),
ainsi que, en outre, un halogène, un groupe alkyle en C₁ à C₄, alkyle en C₁ à C₄ portant 1 à 6 halogènes, alkoxy en C₁ à C₄, alkylthio en C₁ à C₄ et OH au cas où l'un au moins des restes R₂ et R₃ a une définition autre qu'un atome d'hydrogène, de même qu'en outre un groupe alkoxy en C₁ à C₄ et NH₂ au cas où R¹ représente un groupe alkyle en C₁ à C₆ éventuellement substitué,
les substituants des restes éventuellement substitués pouvant être un halogène, un groupe alkyle en C₁ à C₄, halogénalkyle en C₁ à C₄, alkoxy en C₁ à C₄, halogénalkoxy en C₁ à C₄, halogénalkylthio en C₁ à C₄, halogénalkylsulfinyle en C₁ à C₄, halogénalkylsulfonyle en C₁ à C₄, méthylènedioxy ou éthylènedioxy, qui sont éventuellement substitués par du fluor ou du chlore, amino, alkylamino en C₁ à C₄, di(alkyle en C₁ à C₄)amino, acétylamino.

2. Procédé de production des dérivés substitués de 1,2,4-oxadiazoles de formule (I) et de leurs stéréo-isomères, 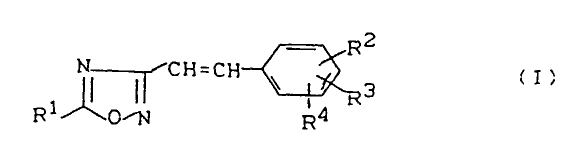 formule dans laquelle
R¹ représente l'hydrogène, un groupe alkyle en C₁ à C₆ qui est substitué le cas échéant par 1 à 6 atomes d'halogènes, cycloalkyle en C₃ à C₆, alkoxy en C₁ à C₆, hydroxy, phényle éventuellement substitué, (alkyle en C₁ à C₄)carbonyle, (alkoxy en C₁ à C₆)carbonyle ainsi qu'un groupe cycloalkyle en C₃ à C₆,
R représente de l'hydrogène, un halogène, un groupe CN, NO₂, alkyle en C₁ à C₆, alkyle en C₁ à C₆ portant 1 à 6 atomes d'halogènes, alkoxy en C₁ à C₆ ou thioalkyle en C₁ à C₆,
R³ représente l'un des restes indiqués pour R,
R⁴ est un groupe SH, un groupe alkoxy en C₁ à C₆ portant 1 à 5 halogènes, un groupe alkylthio en C₁ à C₄ portant 1 à 6 halogènes, un groupe alkylsulfinyle en C₁ à C₆ portant 1 à 6 halogènes, un groupe alkylsulfonyle en C₁ à C₆ portant 1 à 5 halogènes, un groupe phénoxy éventuellement substitué, phénylmercapto éventuellement substitué, phényl-(alkoxy en C₁ à C₆) éventuellement substitué, phényl-(alkylthio en C₁ à C₆) éventuellement substitué, alkylènedioxy en C₁ ou C₂, alkylènedioxy en C₁ ou C₂ portant 1 à 4 halogènes, alkylamino en C₁ à C₆, di(alkyle en C₁ à C₄)amino, acétylamino, benzènesulfonylamino, -CONH₂, -CONH(alkyle en C₁ à C₄), (alkoxy en C₁ à C₄)-carbonyle, (alkyle en C₁ à C₄)-carbonyle, (alkyle en C₁ à C₄)-carbonyloxy, (alkoxy en C₁ à C₄)-(alkyle en C₁ à C₄), (alkyle en C₁ à C₄)-(alkoxy en C₁ à C₄) et hydroxy(alkoxy en C₁ à C₄),
ainsi que, en outre, un halogène, un groupe alkyle en C₁ à C₄, alkyle en C₁ à C₄ portant 1 à 6 halogènes, alkoxy en C₁ à C₄, alkylthio en C₁ à C₄ et OH au cas où l'un au moins des restes R₂ et R₃ a une définition autre qu'un atome d'hydrogène, de même qu'en outre un groupe alkoxy en C₁ à C₄ et NH₂ au cas où R¹ représente un groupe alkyle en C₁ à C₆ éventuellement substitué,
les substituants des restes éventuellement substitués pouvant être un halogène, un groupe alkyle en C₁ à C₄, halogénalkyle en C₁ à C₄, alkoxy en C₁ à C₄, halogénalkoxy en C₁ à C₄, halogénalkylthio en C₁ à C₄, halogénalkylsulfinyle en C₁ à C₄, halogénalkylsulfonyle en C₁ à C₄, méthylènedioxy ou éthylènedioxy, qui sont éventuellement substitués par du fluor ou du chlore, amino, alkylamino en C₁ à C₄, di(alkyle en C₁ à C₄)amino, acétylamino,
caractérisé en ce qu'on fait réagir un dérivé d'amidoxime de formule (II) et des stéréo-isomères de ce dérivé 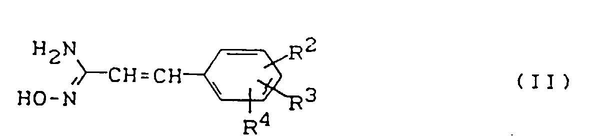 formule dans laquelle
R, R et R⁴ ont les définitions indiquées ci-dessus,
a) avec un orthoester d'acide carboxylique de formule (III)
R¹-C(O-A)₃ (III)
dans laquelle
R¹ a la définition indiquée ci-dessus et
A est un groupe alkyle, autrement un groupe méthyle ou éthyle, ou bien
b) avec un ester d'acide carboxylique de formule (IV) 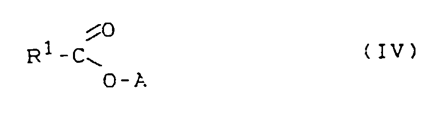 dans laquelle
R¹ et A ont la définition indiquée ci-dessus, ou bien
c) avec des anhydrides d'acides carboxyliques de formule (V)
(R¹-CO)₂O (V)
dans laquelle
R¹ a la définition indiquée ci-dessus, ou bien
d) avec un halogénure d'acide carboxylique de formule (VI) 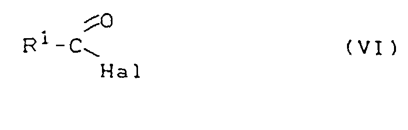 dans laquelle
R¹ a la définition indiquée ci-dessus et
Hal représente un halogène,
en présence d'un diluant et en présence d'un auxiliaire de réaction.

3. Compositions endoparasitizides, caractérisées par une teneur en au moins un dérivé de 1,2,4-oxadiazoles de formule (I) suivant la revendication 1.

4. Utilisation de dérivés de 1,2,4-oxadiazoles de formule (I) suivant la revendication 1 pour la préparation de compositions endoparazidicides.
